# EUROPEAN PATENT SPECIFICATION

(11) **EP 1 755 359 B1**
(45) Date of publication and mention of the grant of the patent: **16.01.2013**
(21) Application number: 05743816.0
(22) Date of filing: 24.05.2005
(51) Int. Cl.: H04R 17/00, A61B 8/00, H04R 31/00, B06B 1/06, G10K 11/00

(54) **SUPERSONIC TRANSDUCER AND MANUFACTURING METHOD THEREOF**
ÜBERSCHALLWANDLER UND HERSTELLUNGSVERFAHREN DAFÜR
TRANSDUCTEUR SUPERSONIQUE ET PROCÉDÉ POUR LA FABRICATION DE CELUI-CI

(30) Priority: 24.05.2004 JP 2004153048
(43) Date of publication of application: 21.02.2007
(73) Proprietor: Olympus Corporation, Tokyo 151-0072 (JP)
(72) Inventor: ADACHI, Hideo, c/o OLYMPUS CORPORATION, Tokyo 1510072 (JP); IDETA, Noriaki, c/o OLYMPUS CORPORATION, Tokyo 1510072 (JP); ONOZUKA, Kazuhisa, c/o OLYMPUS CORPORATION, Tokyo 1510072 (JP)
(74) Representative: von Hellfeld, Axel
(86) International application number: PCT/JP2005/009475
(87) International publication number: WO 2005/115054

(56) References cited:
- EP-A2- 0 190 948
- JP-A- 6 086 397
- JP-A- 7 050 898
- JP-A- 2002 124 712
- JP-A- 2002 135 895
- JP-A- 2003 310 608

## Description

### Technical Field

The present invention relates to an array type ultrasonic transducer for use in an electronic scanning ultrasonic wave diagnosis apparatus.

### Background Art

In recent years, an ultrasonic wave diagnosis method has been widely propagated for diagnosing the internal human body by imaging an echo signal obtained by irradiating an ultrasonic wave within the abdomen by using an ultrasonic endoscope. For this, it is necessary to insert, into the abdomen, an insertion part equipped with an ultrasonic transducer on the tip thereof for generating an ultrasonic wave and receiving an ultrasonic wave reflected within the abdomen in order to obtain an image in the inside of the abdomen by using an ultrasonic wave diagnosis method.

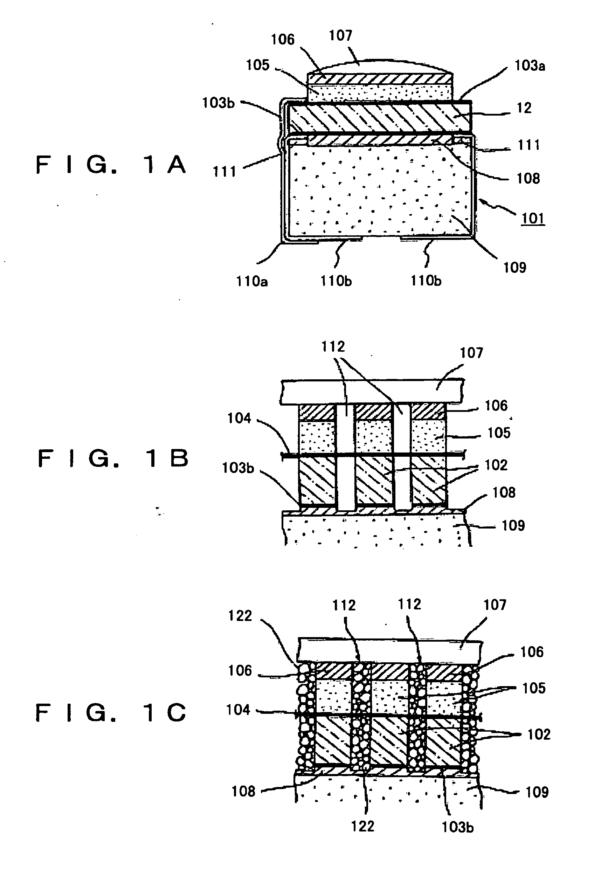

Fig. 1A is a cross-sectional diagram of a conventional array-type ultrasonic wave probe. Fig. 1B is a side cross-sectional diagram of the array-type ultrasonic probe shown by Fig. 1A in the perpendicular direction with its part being enlarged.

Referring to Figs. 1A and 1B, the array type ultrasonic transducer 101 primarily comprises piezoelectric elements 102, electrodes 103a and 103b, a flexible board 104, matching layers 105 and 106, an acoustic lens 107, an insulating layer 108, a dumping layer 109, flexible leads 110a and 110b, adhesive 111 and a gap area 112.

An array type piezoelectric element 102 is comprised by thinly (in a small width) slicing in the vertical direction a piezoelectric element plate (e.g., PZT allowing polarization in the direction of thickness of the plate), and by arraying them in parallel with each slice being slightly separated from the adjacent one. A vapor deposited silver, for example, is applied to both surfaces of the piezoelectric element 102 in the thickness direction to form the electrodes 103a and 103b. The surface of the electrode 103a on the side of transmitting and receiving ultrasonic waves is disposed as a ground-side electrode. The conductive plate shape flexible electrode 104 maintains the conductivity of each electrode 103a.

Furthermore, formed in a layer on the flexible electrode 104 are the first acoustic matching layer 105 and the second acoustic matching layer 106 which are thinly formed by the same feature as each of the piezoelectric elements 102 and are adhered. Formed on the upper surface of the second acoustic matching layer 106 is the acoustic lens 7 having a convex surface at the center of the longitudinal direction of each of the piezoelectric elements 102.

Meanwhile, featured on the surface of the electrode 103b of each of the piezoelectric elements 102 is the insulating layer 108 which composes an insulating member. The dumping layer 109 is fixed onto the insulation layer 108. Each transducer element is formed using this method.

The electrode 103b is drawn out from both sides of the dumping layer 109 to the rear side by the flexible lead 110b. The ground-side electrode 103a is also drawn out to the rear side of the dumping part 109 by the flexible lead 110a.

The insulation layer 108 is fixed onto the dumping part 109 by an adhesive 111, such as an epoxy resin.

The first acoustic matching layer 105 and second acoustic matching layer 106 are set up with an intermediate value of acoustic impedance between the piezoelectric element 102 and the inner wall of the abdomen. This configuration makes it possible to transmit an ultrasonic wave from (or received by) the piezoelectric elements 102 efficiently (or by minimizing a reflection) with respect to the inner wall of an abdomen to which the front (or the top) surface of the acoustic lens 107 contacts. The configuration further makes double-layered acoustic matching layers 105 and 106, thereby enabling a further smooth matching of acoustic impedance.

An ultrasonic wave is transmitted from each of the piezoelectric elements 102 being excited by an ultrasonic wave pulse applied to both of the electrodes 103a and 103b. Here, the dumping layer 109 is disposed for preventing degraded resolution as a result of an ultrasonic wave (reflected on the rear surface of the dumping layer 109) being divided by dumping the ultrasonic wave transmitted out toward the rear surface thereof.

The dumping layer 109 can be configured to possess an adequate attenuation function by making its thickness large enough for use within the abdomen. However, because the dumping layer 109 should have a thickness enough to attenuate ultrasonic wave when the dumping layer 109 is used within the abdomen, a small thickness is required.

The dumping layer 109, accordingly, is made from a tungsten powder dispersed in an epoxy resin, a silicone resin, a vinyl chloride resin, and so forth. Here, the tungsten powder is dispersed in a resin so that the dispersed amount thereof is about 95-weight percent, thereby accomplishing approximately satisfactory attenuation.

In the case of using the dumping layer 109 of the above noted material, the ultrasonic wave transmission/reception surface side is set as the ground electrode while the dumping layer 109 side is set as the signal applied electrode for ensuring the safety of the human body because the electrical resistance of the material is low. Because of this, if the dumping layer 109 comes in directly contact with the signal applied electrode side, a failure occurs in which each ultrasonic transducer element (that is divided) in array is conducted with low impedance of a member forming the dumping layer 109. Therefore, the dumping layer 109 is insulated from the signal applied electrode 103b by the insulating layer 108.

Incidentally, each ultrasonic transducer can be equipped with a gap area between adjacent elements in order to prevent crosstalk between each element. Furthermore, it is possible to prevent such crosstalk more securely by not only equipping a gap area between the individual elements, but also equipping a gap area 112 for three layers including the first acoustic matching layer 105 and second acoustic matching layer 106.

Note that the ultrasonic transducer, separated by the gap area 112, is formed in the following manner. For example, at the beginning, a piezoelectric element plate and the first and the second acoustic matching plates being stacked are integrated. This piezoelectric element plate includes electrodes formed on both surfaces thereof, and is fixed onto the insulating layer 108. The next is to cut it using dicing saw so as to separate the electrode 103b by cutting into a part of the insulating layer 108. In this case the flexible printed circuit (FPC) 104 is formed in a manner to make contact with the ground electrode 103a after the cutting.

In a thusly configured conventional ultrasonic transducer, the adjacent piezoelectric elements 102 are separated from each other (including the matching layers 105 and 106), and therefore crosstalk is adequately prevented. However, each element is arrayed in a thin and long state, and the both ends are unsupported, therefore having a severe shortfall of mechanical strength.

Another problem includes the issue that moisture may seep into the gap area 112 due to humidity, for example, may remain therein for an extended period of time. This causes the silver on the electrodes 103a and 103b (applied onto both surfaces of each of the piezoelectric elements 102) to migrate, creating a risk of a degraded function of transmitting and receiving ultrasonic waves and further deterioration, possibly causing a shorting in a severe case.

Accordingly, patent document 1 has proposed a technique for preventing crosstalk and moisture from seeping into by filling the gap area between the elements with hollow members 122 made of hollow glass balls as shown in Fig. 1C in order to improve the above described shortfall.
document 1: Laid-Open Japanese Patent Application Publication No. Sho 60-89199

### Disclosure of Invention

Some or all of the above problems are overcome by an ultrasonic transducer comprising the features of claim 1 and/or a production method for an array type ultrasonic transducer comprising the features of claim 16.

According to the present invention, an ultrasonic transducer is configured comprising an transducer element (which includes a piezoelectric resonator oscillating for emitting an ultrasonic wave and one or more acoustic matching layer(s)), and an acoustic lens (wherein a gap area between the adjacent transducer elements is filled with the same constituent material as that of the acoustic lens).

The ultrasonic transducer according to the present invention is also configured to cover an external surface thereof with the same constituent material as that of the acoustic lens.

The ultrasonic transducer according to the present invention is also configured in a manner such that a constituent material of the acoustic lens is a gradient material.

The ultrasonic transducer according to the present invention is also configured in a manner such that the gradient material is a dispersing inorganic fine particulate powder in a silicone resin, with the filling density of the inorganic fine particulate powder decreasing with the direction from an oscillation generation surface of the piezoelectric element to the bordering surface between the acoustic lens and acoustic matching layer.

The ultrasonic transducer according to the present invention is also configured in a manner such that the inorganic fine particulate powder includes at least one of the following: tungsten, tungsten oxide, aluminum oxide and zirconium oxide.

The ultrasonic transducer according to the present invention is also configured in a manner such that particulates, each of which has a hollow structure and a smaller specific gravity than a silicone resin, are dispersed in the silicone resin for the gradient material with the filling density of the inorganic fine particulate powder decreasing with the direction from an oscillation generation surface of the piezoelectric element to the bordering surface between the acoustic lens and acoustic matching layer.

The ultrasonic transducer according to the present invention is also configured in a manner so that a glass material constitutes the particulate.

The ultrasonic transducer according to the present invention is also configured in a manner such that a polymer material constitutes the particulate.

The ultrasonic transducer according to the present invention is also configured in a manner to make a thin film layer with a corrosion resistance property or humidity resistance property intervene between a surface where a material, which forms the acoustic lens and fills between the transducer elements and the transducer element contact with each other.

The ultrasonic transducer according to the present invention is also configured in a manner such that the thin film layer includes a nano-coating layer containing an inorganic compound component.

The ultrasonic transducer according to the present invention is also configured in a manner such that the inorganic compound component includes at least one of the following: silicone, titanium, and zirconium.

The ultrasonic transducer according to the present invention is also configured to make a thin film layer having a corrosion resistance property or humidity resistance property exist on a covered surface of the ultrasonic transducer that is covered with the same constituent material as that of the acoustic lens.

The ultrasonic transducer according to the present invention is also configured in a manner such that the thin film layer includes a nano-coating film containing an inorganic compound component.

The ultrasonic transducer according to the present invention is also configured in a manner such that the nano-coating film contains at least one of the following: silicone oxide, titanium oxide, and zirconium oxide.

The ultrasonic transducer according to the present invention is also configured to form a silver nano-coating film on a surface of the thin film layer.

According to the present invention, a production method for an array type ultrasonic transducer comprises : a junction process for joining an electrode surface of a flexible board to a piezoelectric transducer, so as to connect an electrode of the piezoelectric transducer to the electrode surface of the FPC; an acoustic matching layer junction process for joining one or more acoustic matching layers to a joined body joined by the junction process; a backing material layer junction process for joining a layered body generated by the acoustic matching layer junction process onto a backing material retaining the layered body; a dicing process for applying a dicing process to the layered body; a ground wire connection process for connecting a common ground wire for making a ground side of a piezoelectric transducer element formed by the dicing process a common potential; a primer treatment process for applying a primer treatment to a groove formed by the dicing process; and a resin precursor curing process for fixing the layered body obtained as a result of the primer treatment process to a preconfigured mold, making a resin precursor, which becomes covered parts of an acoustic lens, of a filling material for the groove and of an outside of the layered body, contact with the part applied by the primer treatment, and curing the resin precursor.

The production method for an array type ultrasonic transducer, according to the present invention, is also contrived in a manner such that the primer treatment process is followed by carrying out a nano-coating film layer forming process for forming a nano-coating film layer for the part applied by the primer treatment.

The production method for an array type ultrasonic transducer according to the present invention is also contrived in a manner such that the resin precursor curing process is followed by carrying out a nano-coating film layer forming process for forming a nano-coating film layer on the cured resin precursor body.

The production method for an array type ultrasonic transducer according to the present invention is also contrived in a manner such that the resin precursor contains a silicone elastomer precursor and a dilute solvent.

According to the present invention, a production method for an array type ultrasonic transducer comprises : an transducer element forming process for forming an transducer element, including a piezoelectric resonator for emitting an ultrasonic wave and one or more acoustic matching layers; and a resin precursor curing process for curing a resin precursor by fixing a layered body, which is obtained by the transducer element forming process, to a preconfigured mold and making the layered body contact with the resin precursor which covers parts of an acoustic lens with a filling material in between the transducer elements and of an outside of the layered body.

An array type ultrasonic transducer according to the present invention is equipped on an ultrasonic wave endoscope apparatus.

An array type ultrasonic transducer produced by the production method according to the present invention is equipped on an ultrasonic wave endoscope apparatus.

### Brief Description of Drawings

Fig. 1A is a cross-sectional diagram of a conventional array type ultrasonic wave probe;
Fig. 1B is a side cross-sectional diagram of the array type ultrasonic wave probe shown by Fig. 1A in the perpendicular direction with its part being enlarged;
Fig. 1C shows how hollow members made of glass balls being hollow are filled in a gap area between elements of a conventional array type ultrasonic wave probe;
Fig. 2A is a diagram showing an external view (i.e., a diagonal side view) of an array type ultrasonic transducer 1 according to a first embodiment;
Fig. 2B is a diagram showing an external view (i.e., a long side view) of the array type ultrasonic transducer 1 according to the first embodiment;
Fig. 2C is a diagram showing an external view (i.e., a short side view) of the array type ultrasonic transducer 1 according to the first embodiment;
Fig. 3A is a diagram observed from a side of the array type ultrasonic transducer 1 according to the first embodiment;
Fig. 3B is a cross-sectional diagram observed from a perpendicular direction of Fig. 3A;
Fig. 3C is a cross-sectional diagram observed from an upper direction of Fig. 3A;
Fig. 4A is a diagram (i.e., a long side cross-sectional view) showing an internal configuration of the array type ultrasonic transducer 1 according to the first embodiment;
Fig. 4B is a diagram (i.e., a short side cross-sectional view) showing an internal configuration of the array type ultrasonic transducer 1 according to the first embodiment;
Fig. 5A is a diagram (i.e., a long side cross-sectional view) showing an array type ultrasonic transducer according to the second embodiment;
Fig. 5B is a diagram (i.e., a part of a short side cross-sectional view) showing the array type ultrasonic transducer according to the second embodiment;
Fig. 6 is a diagram showing a structure of a protection film according to the second embodiment;
Fig. 7A is a diagram (i.e., a long side cross-sectional view) showing an array type ultrasonic transducer according to the third embodiment;
Fig. 7B is a diagram (i.e., a part of a short side cross-sectional view) showing the array type ultrasonic transducer according to the third embodiment;
Fig. 8 is a diagram showing an array type ultrasonic transducer according to the fourth embodiment;
Fig. 9 is a diagram showing an array type ultrasonic transducer according to the fifth embodiment;
Fig. 10A is a diagram (i.e., a long side cross-sectional view) showing an internal configuration of an array type ultrasonic transducer 1 according to the sixth embodiment;
Fig. 10B is a diagram (i.e., a part of a short side cross-sectional view) showing the array type ultrasonic transducer according to the sixth embodiment;
Fig. 11A is a diagram showing a condition of a production process according to the seventh embodiment (part 1);
Fig. 11B is a diagram showing a condition of the production process according to the seventh embodiment (part 2);
Fig. 11C is a diagram showing a condition of the production process according to the seventh embodiment (part 3);
Fig. 11D is a diagram showing a condition of the production process according to the seventh embodiment (part 4); and
Fig. 11E is a diagram showing a condition of the production process according to the seventh embodiment (part 5).

### Best Mode(s) for Carrying Out the Invention

### <First Embodiment>

In the conventional method, an acoustic lens is adhered to a matching layer by using an adhesive, resulting in an excessive amount of the adhesive flowing into the gap area 112. There is a fluctuation in the amount of the adhesive flowing from a gap area 112 to the next, with the fluctuation causing a variation of an ultrasonic wave characteristic.

A description of the present embodiment refers to an ultrasonic transducer that integrally forms an acoustic lens and a groove-filling material by using the same material.

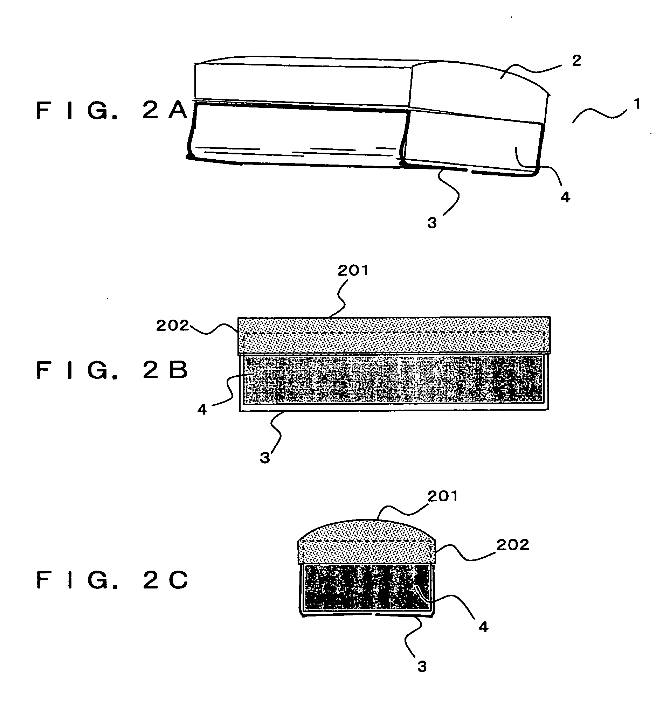

Figs. 2A through 2C show external views of the array type ultrasonic transducer 1 according to the present embodiment. Fig. 2A is an external diagnal side view of the array type ultrasonic transducer 1 according to the present embodiment. Fig. 2B is an external long side view of the array type ultrasonic transducer 1 according to the present embodiment. Fig. 2C is an external short side view of the array type ultrasonic transducer 1 according to the present embodiment.

Referring to Figs. 2A through 2C, the upper side of a backing material 4 is covered with an acoustic lens and external cover 2 (noted as "acoustic lens / external cover 2" hereinafter for this compound component). Across the side and bottom surfaces of the backing material 4 is equipped with a flexible printed circuit (FPC) 3. Note that on the surface of the FPC 3 is laid a cable for transmitting and receiving a signal, except that it is omitted in these drawings. A numerical 201 indicates a part forming an acoustic lens of the acoustic lens / external cover 2. A numerical 202 indicates a part forming an external cover (or a side resin film).

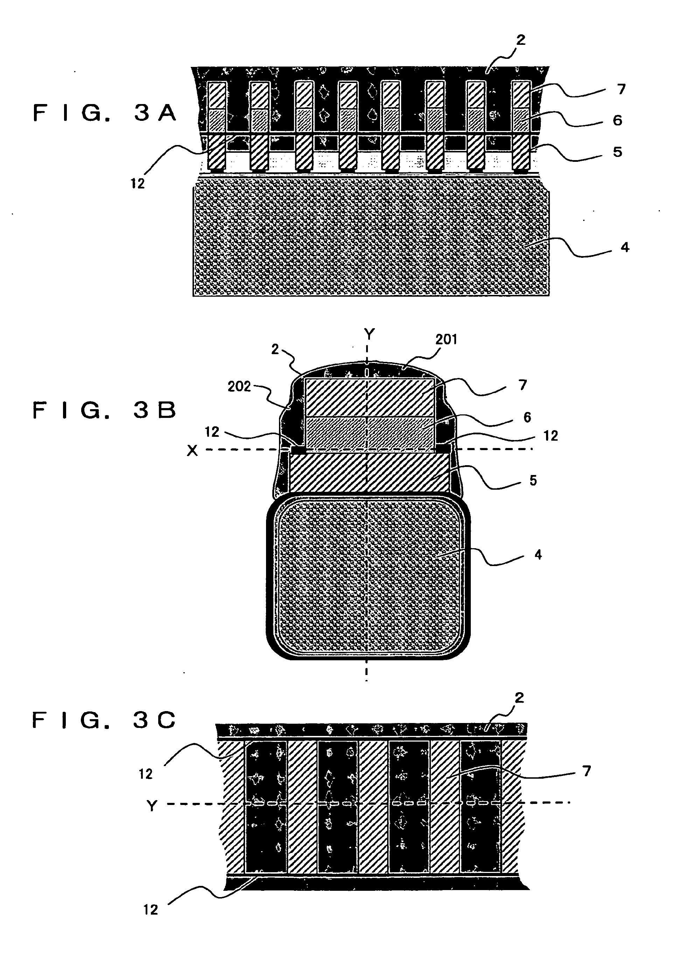

Figs. 3A through 3C show diagrams of observations, from their respective directions, of the array type ultrasonic transducer 1 according to the present embodiment. Fig. 3A is a diagram observing from a side. Fig. 3B is a cross-sectional diagram observed from a perpendicular direction of Fig. 3A. Fig. 3C is a cross-directional diagramof Fig. 3A observed from above, corresponding to a cut plane represented by the dotted line X shown in Fig. 3B.

The present embodiment is configured such that an transducer element (or it is also simply called "element" herein) is constituted by a piezoelectric resonator (i.e., a piezoelectric element) 5, a first acoustic matching layer 6 and a second acoustic matching layer 7. The piezoelectric element 5 oscillates upon receiving a voltage signal, generating an ultrasonic wave. When emitting an ultrasonic wave, as is, into air, water or a living body, the ultrasonic wave is not effectively emitted as a result of being reflected by the boundary face because there is an acoustic impedance difference between the piezoelectric resonator and other bodies such as air, water or a living body. Equipping the first acoustic matching layer 6 and second acoustic matching layer 7 with appropriate materials suppresses the ultrasonic wave from being reflected or attenuated by the boundary face, thereby enabling an effective emission of the ultrasonic wave.

The backing material 4 is used for retaining the piezoelectric resonator 5 on the back (i.e., backing the piezoelectric resonator 5). The backing material 4 is also used for obtaining a wide band ultrasonic wave by attenuating an ultrasonic transducer. This, on the other hand, reduces sensitivity proportinately with bandwidth.

The upper part of the backing material 4 is equipped with a plurality of elements. The upper part of the left and right ends of the piezoelectric resonator 5 is placed a common groundwire 12 which is placed across the elements. Note that a dotted line Y is drawn in Figs. 3B and 3C. The following drawings are cross-sectional diagrams cut by the dotted line Y.

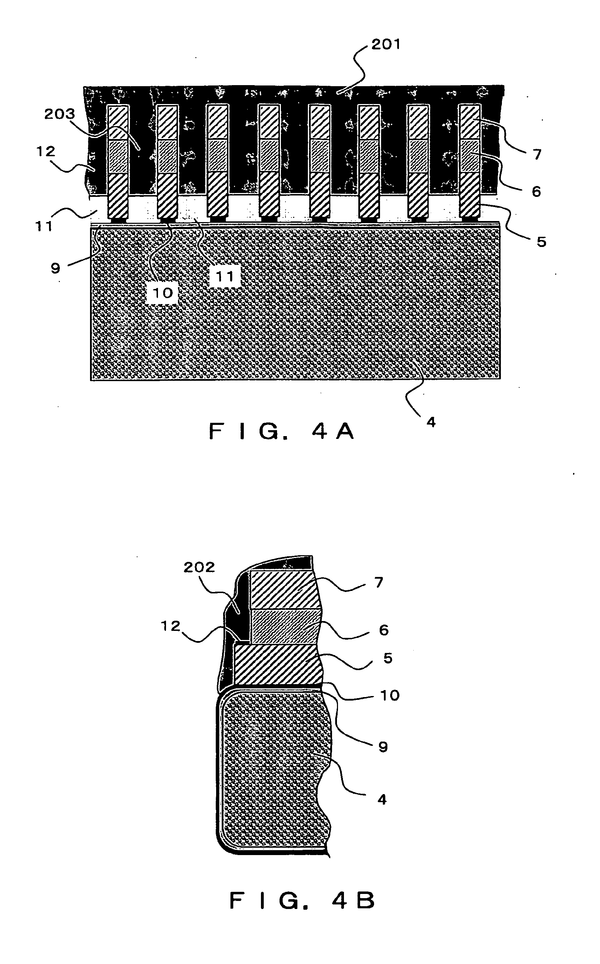

Figs. 4A and 4B are diagrams showing an internal configuration of the array type ultrasonic transducer 1 according to the present embodiment. Fig. 4A is a long side cross-sectional view. Fig. 4B shows a part of a short side cross-sectional view. The lower surface of the piezoelectric element 5 is equipped with a wiring electrode 10. The upper surface of the backing material 4 is equipped with a FPC 9, which is wired in a stripe. The structure is designed such as to adhere, by using an adhesive 11, the wiring on the board 9 with the wiring electrode 10 on the lower surface of the piezoelectric element 5. As described above, a plurality of elements is placed on the backing material 4, and the acoustic lens / external cover 2 exists in a manner to cover these elements.

The acoustic lens / external cover 2 has primary parts 201, 202 and 203. The part 201 forms an acoustic lens. The part 202 forms an external cover (or a side surface resin film). The part 203 is used as a groove filling part which fills groove existing between elements. The parts 201, 202 and 203 are all constituted by the same resin material. This material needs to be selected from ones with a large attenuation effect of an ultrasonic wave. Also necessarily considered is the material-specific sound propagation speed. Considering this, the present embodiment is configured to use a silicone resin (e.g., "ELASTOSIL®, "manufactured by Wacker Asahikasei Silicone Co., Ltd.).

Meanwhile, fixing not only the groove 203 but also the external cover 202 with the silicone resin results in increasing the mechanical strength of the element. An integral forming of the acoustic lens and groove filling by using the same material eliminates the use of an adhesive, thereby preventing variations in an ultrasonic wave characteristic as a result of uneven flow of an excessive amount of adhesive in between elements.

### <Second Embodiment>

A description of the present embodiment refers to the array type transducer according to the first embodiment further using a protective film 13 which is incidentally a film (i.e., a nano coating film) constituted by particulates of a nanometer size.

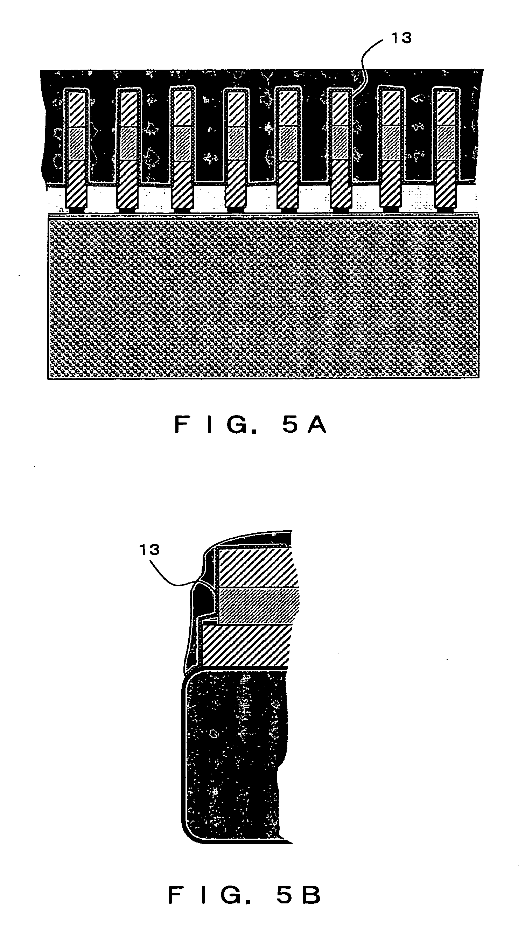

Figs. 5A and 5B show an array type ultrasonic transducer 1 according to the present embodiment. Fig. 5A is a long side cross-sectional diagram. Fig. 5B is a part of a short side cross-directional diagram. The configuration shown by Fig. 5B is such that the element, adhesive layer 11 and common ground wire 12 are covered with the protective film 13 and further covered with the acoustic lens and external cover .

The array type ultrasonic transducer 1 is a part of a medical instrument for use in an ultrasonic wave endoscope, thus necessitating a cleaning and a sterilization before or after a use of the ultrasonic wave endoscope. A disinfectant used here, however, penetrates a silicone resin forming the acoustic lens / external cover 2. Consequently, there is a possibility of the penetrated disinfectant seeping into the lowest part of a silicone resin layer where the piezoelectric element 5 exists. It is also possible for vapor to penetrate under a pressurized condition.

In these cases, a piezoelectric resonator may be shorted or corroded by the penetrated disinfectant or vapor. Also conceivable is the case that a normal diagnosis image cannot be obtained.

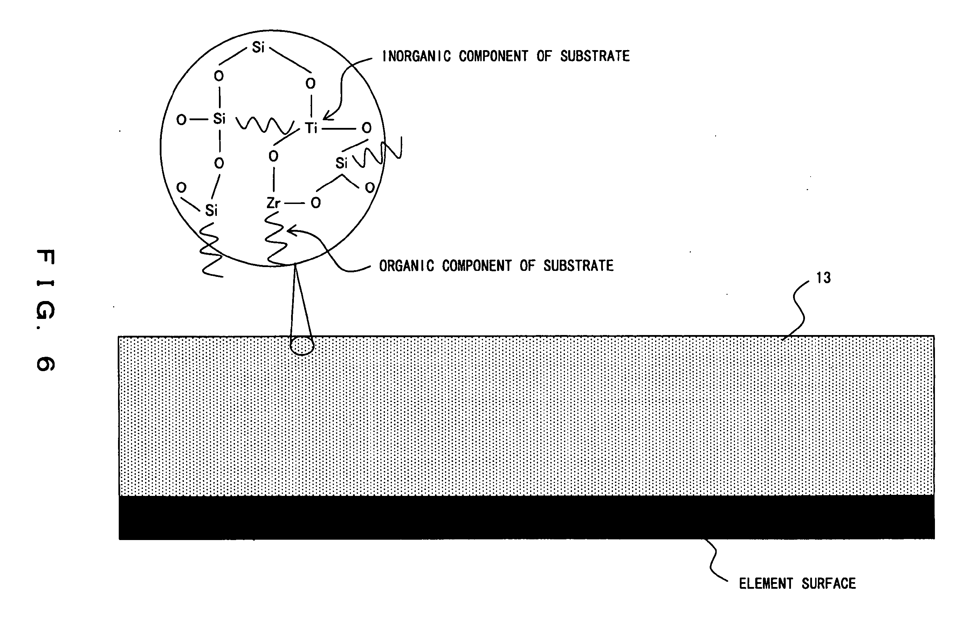

Accordingly, placing a thin film layer (i.e., a protective film) having a corrosion resistance property and/or a humidity resistance property between a silicone resin and an element (i.e., a boundary face) makes it possible to protect a piezoelectric resonator from the disinfectant or vapor penetrating a silicone resin. The present embodiment is configured to use a coating thin film layer. This is described in association with Fig. 6.

Fig. 6 shows a configuration of a protective film (i.e., product name: x-protect DS 3010, produced by NANO-X GmbH) according to the present embodiment. The protective film 13 is constituted by inorganic components of silicone (Si), zirconium (Zr) and titanium (Ti), oxygen (O), and other organic components (i.e., polymer compounds), and is of a mesh structure, as shown in Fig. 6. This structure is obtained by hydrolyzing a metal alcoxide compound such as silicone (Si), zirconium (Zr) and titanium (Ti).

As shown in Fig. 6, the organic components as substrate exist in a manner to twine the mesh structure of the inorganic components. While the structure is formed in mesh across the entirety of the film, an area in which the mesh structure of the inorganic components is not twined by the organic components is formed by a production method. Having been spared the organic component twining, the organic components exist as nano particulates free from the mesh structure. Therefore, whether the structure of the nano-coating film is made of nano-particulates (which also have a mesh structure) or a mesh structure twined by the organic components extending across the entirety of the film can be controlled, for example, by allocating production conditions, such as a difference in heating condition, methods of hydrolysis, PH adjustments, and so forth. Note that an inorganic compound component of nanometer size may be constituted of silicone, titanium or zirconium, or may be a combination thereof; it also may be constituted of silicone oxide, titanium oxide or zirconium oxide, or a combination of those, thereof. A protective film having these inorganic compounds as the components possesses a corrosion resistance property and/or humidity resistance property.

Alternatively, a silver nano-coating maybe formed on the surface of the formed thin film layer described above. That is, referring to Figs. 5A and 5B, a silver nano-coating film may be formed on the boundary face between the protective film 13 and a silicone resin. The characteristics of silver include an antibacterial effect because a microscopic organism is negatively (-) charged and therefore its cell is destroyed when contacting with a positively (+) charged silver ion, and hence the microscopic organism is exterminated.

The silver nano-coating film comprises the entirety of the film by dispersing silver particulates of a nanometer size in an imide resin compound, for example, where the imide resin twining the mesh of the nanometer size silver particulates has a mesh structure.

As described above, use of the nano-coating film, having a component such as silicone, titanium or zirconium, enables the accomplishment of an array type ultrasonic transducer possessing a corrosion resistance property and/or a humidity resistance property. Furthermore, the forming of the silver nano-coating film makes it possible to create an array type ultrasonic transducer possessing an antibacterial effect. This configuration enables the safer use of the ultrasonic endoscope for the abdomen.

### <Third Embodiment>

The second embodiment forms a layer of a protective film on the boundary face between a silicone resin and an element. Comparably, the present (third) embodiment is configured to cover the surface of a silicone resin, that is, the acoustic lens / external cover 2, with a protective film, thereby preventing a penetration of a disinfectant or vapor into the silicone resin.

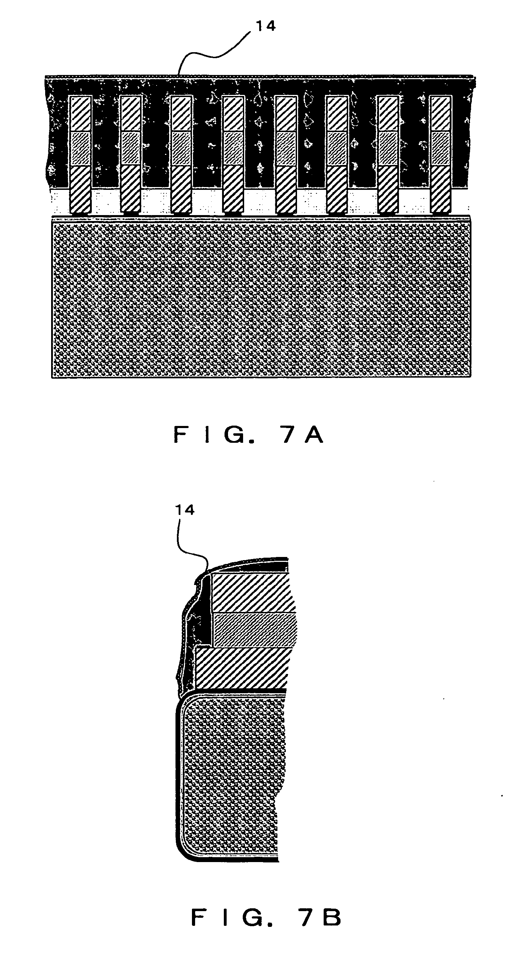

Figs. 7A and 7B show an array type ultrasonic transducer according to the present embodiment. Fig. 7A is a long side cross-sectional diagram. Fig. 7B is a diagram showing a part of a short side cross-sectional diagram. This array type ultrasonic transducer is the one shown in Fig. 4 with the surface of its acoustic lens / external cover 2 contacting the exterior thereof (i.e., the outside surface) being covered with the protective film 14 as described above for the second embodiment.

The present embodiment is configured to comprise the protective film (i.e., the thin film layer) 14, which has a corrosion resistance property and a humidity resistance property, by nano-particulates comprising an inorganic compound component as in the second embodiment. A component of nano-particulates comprising an inorganic compound component may be silicone, titanium or zirconium, or may be a plurality thereof as in the second embodiment. It may also be silicone oxide, titanium oxide or zirconium oxide, or may be a plurality thereof. A silver nano-coating may be formed on a surface of the thin film layer as in the second embodiment.

Such a configuration prevents a disinfectant or vapor from penetrating the silicone resin structuring the acoustic lens / external cover 2; it therefore prevents the piezoelectric element from being shorted or corroded. Note that a forming of protective film layers in between the acoustic lens / external cover 2 and element, and an externally contacting surface of the acoustic lens / external cover 2, respectively, as a combination of the second and third embodiment, further improves the corrosion resistence and humidity resistance properties.

### <Fourth Embodiment>

A description of the present fourth embodiment refers to the case of using a gradient material (i.e., a material developing a new function by a gradient distribution of ingredient composition and microscopic organization internally to the material) as a material of the acoustic lens / external cover 2.

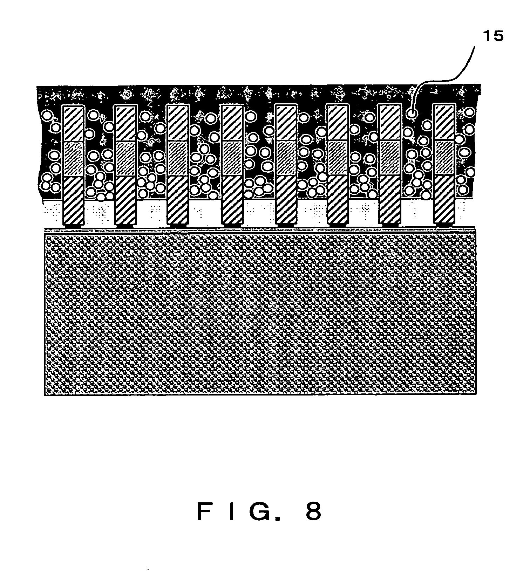

Fig. 8 shows an array type ultrasonic transducer according to the present embodiment. Fig. 8 shows the case of using hollow-structured inorganic fine particulate powder 15 in between each of elements of the array type ultrasonic transducer shown in Fig. 4. The inorganic fine particulate powder comprises ingredients including, at least, tungsten, tungsten oxide, aluminum oxide or zirconium oxide. The inorganic fine particulate of the powder has a hollow structure. The inorganic fine particulate may be made of a hollow glass material or hollow polymer material.

Such a hollow particulate uses a material of a specific gravity smaller than a silicone resin, because it is necessary to make the hollow particulate within a silicone resin rise by buoyancy. As a result, it is possible to make the hollow particulates disperse in-between elements filled with a silicone resin with a gradient dispersion density.

The structure is configured such that the dispersion density of the hollow particulates becomes increases (i.e., a filling ratio of the hollow particulates becomes decreases) in proportion with a distance from an ultrasonic wave emission surface of the piezoelectric resonator. That is, as the hollow particulate comes closer to the piezoelectric resonator, the dispersion density of the hollow particulates becomes decreases (i.e., a filling ratio of the hollow particulates becomes increases). Comparably, as the hollow particulate goes away from the piezoelectric resonator (i.e., as it goes upward of the element), a dispersion density of the hollow particulates becomes increases (i.e., a filling ratio thereof becomes decreases). Between elements, crosstalk tends to occur most between piezoelectric resonators on the lower side of the element. Due to this, making a filling ratio of the hollow particulates high in the neighborhood of the piezoelectric resonator enables effective suppression of the crosstalk.

The hollow particulates are dispersed in-between elements, with the dispersion density being increased toward the upper part of the elements (i.e., a filling ratio of the hollow particulates is decreased toward the upper part of the elements). However, the dispersion is to be limited in-between elements. That is, care is taken to prevent the hollow particulates from overflowing from between the elements and reaching at the part 201 forming the acoustic lens. The reason is that an ultrasonic wave characteristic is degraded if the hollow particulates disperse to the acoustic lens 201 as a result of controlling a mixing ratio of the hollow particulates.

The hollow particulates are also dispersed in a gradual gradient from the lower part to upper part of the element so as to avoid a rapid change of the dispersion density, because a rapid change of the dispersion density causes such a part to become a boundary face resulting in reflecting an ultrasonic wave.

Incidentally, the size of the hollow particulates used is between ones micrometer and ten micrometers, although it depends on the size of a groove width or depth. This is not important, however.

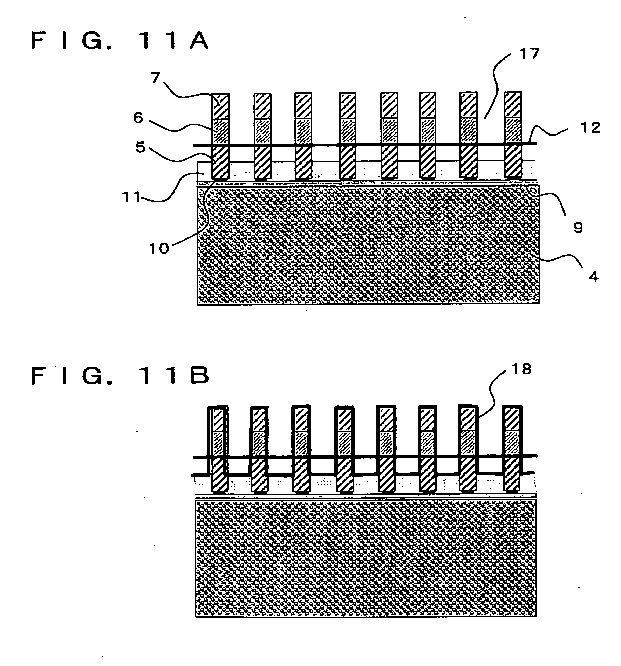

It is also possible to use hollow particulates of a small specific gravity and of a large specific gravity by mixing them together. In such a case, a use of a later described production process to be shown in Fig. 11A through 11E distributes hollow particulates of a small specific gravity first in a deeper part of the groove and ones of a large specific gravity in a shallower part of the groove. This configuration makes it possible to reduce crosstalk.

The above-described configuration makes it possible to effectively prevent crosstalk between the adjacent elements and further improve mechanical strength.

### <Fifth Embodiment>

The present (fifth) embodiment is a modified example of the fourth embodiment, above, which uses hollow particulates, whereas the present embodiment uses non-hollow particulates (e.g., content-filled particulates).

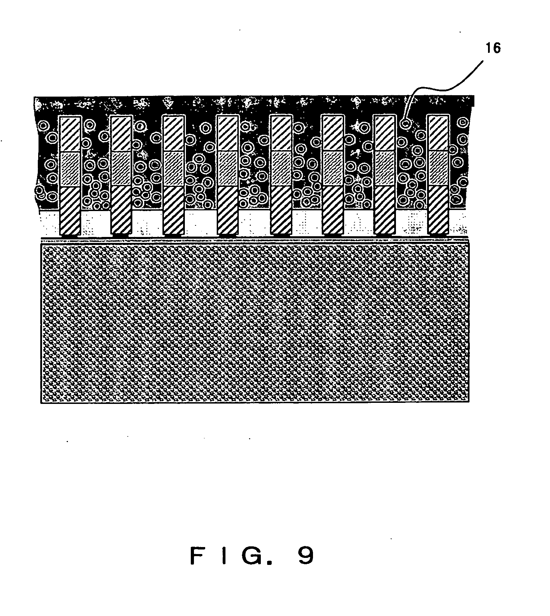

Fig. 9 shows an array type ultrasonic transducer according to the present embodiment. The configuration shown by Fig. 9 uses content-filled particulates 16 in place of the hollow particulates 15 (which are used in Fig. 8) . This configuration makes it possible to obtain a similar effect as in the fourth embodiment.

The above-described configuration makes it possible to effectively prevent crosstalk between adjacent elements and further improve mechanical strength.

### <Sixth Embodiment>

A description of the present (sixth) embodiment refers to the situation in which an array type ultrasonic transducer comprising elements, each of which is applied by dicing from the front and rear surfaces, respectively, is used.

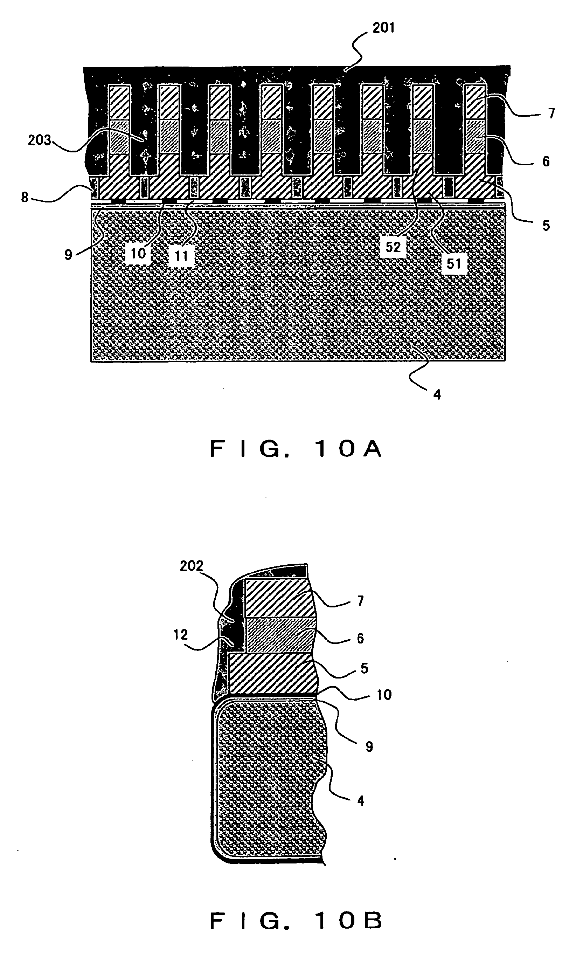

Figs. 10A and 10B show an array type ultrasonic transducer according to the present embodiment. Fig. 10A is a long side cross-directional diagram. Fig. 10B is a diagram showing a part of a short side cross-directional diagram. In the configuration shown by Figs. 10A and 10B, the lower part 51 of a piezoelectric element 5 is larger than the upper part 52 thereof, and there are bottom groove filling parts 8 between the lower parts 51 of the adjacent piezoelectric resonators, as compared to the configuration shown in Figs. 4A and 4B. Note that the filling parts between the upper parts of elements are called the upper filling parts, as opposed to the bottom groove filling parts 8.

The production process for the configuration shown by Figs. 10A and 10B is first to comprise a joined body by joining the piezoelectric element 5, first acoustic matching layer 6 and second acoustic matching layer 7, followed by dicing the surface on the piezoelectric element 5 side for forming a bottom groove (which is the part corresponding to the bottom groove filling part 8), and by dicing the surface on the second acoustic matching layer 7 side for forming an upper groove (which is the part corresponding to the upper filling part) in a larger width than the bottom dicing width.

Such a design enables the transducer element to resist falling because the width on the bottom side is wider, thereby making it possible to improve mechanical strength. Note that the element of the form used for the present embodiment can be used for any configuration of the first through fifth embodiments.

### <Seventh Embodiment>

A description of the present (seventh) embodiment refers to a production method for the array type ultrasonic transducer described for the above described embodiment.

Figs. 11A through 11E collectively show a condition of a production process of the array type ultrasonic transducer according to the present embodiment. The next description is of the production process for the array type ultrasonic transducer according to the present invention while referring to these drawings. Note that any applicable drawing corresponding to the steps S1 through S3 among the following processes is omitted.

S1: First is to connect the electrode 10 of the piezoelectric resonator 5 to the electrode surface of the flexible printed circuit board 9 (what is joined is called a joined body).

S2: Next is to make the first acoustic matching layer 6 and second acoustic matching layer 7 join the joined body, followed by placing the thusly obtained layered body on the backing material by joining it thereon.

S3: Next is to apply a dicing process to the layers of the joined layered body constituted by the flexible printed circuit board 9, piezoelectricresonator5, first acoustic matching layer 6 and second acoustic matching layer 7. As a result of the dicing process, a plurality of elements and the grooves 17 which are generated by the dicing process in between the elements are formed.

S4 : Next is to connect a common ground wire 12 for making, as a common electrode, the ground electrodes of the piezoelectric transducer elements formed by the dicing (refer to Fig. 11A).

S5: Following that is to apply a primer treatment to the dicing grooves 17, in order to improve the adhesiveness to a resin precursor adhering in a later described process. The primer treatment may be carried out by dipping the above described joined layered body in a primer treatment fluid, for example, followed by blowing it away by a spray gun or by using another known method. As a result of the treatment, the surface of the diced joined body is covered with a primer treatment film 18 (refer to Fig. 11B).

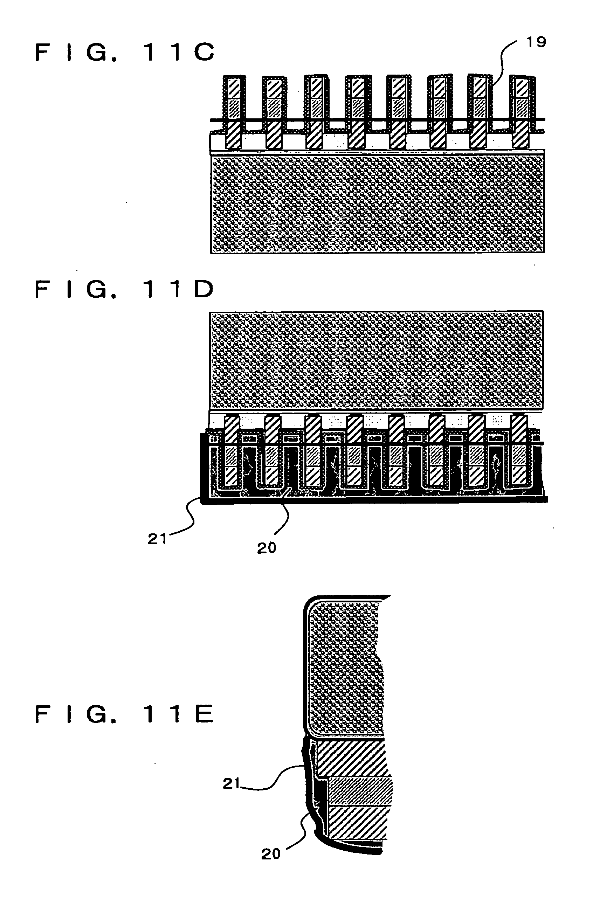

S6: Next, possibly forming a protective film using the nano-coating film 19 (i.e., product name: x-protect DS 3010, produced by NANO-X GmbH) in the case of the second embodiment (refer to Fig. 11C). The forming of the protective film 19 may be carried out by dipping the diced joined layered body, followed by blowing it away by a spray gun, among other blowing instruments, as in the same manner as forming the primer treatment film 18 by the primer treatment, or may be by using another known method. Following the forming of the protective film 19, a primer treatment is applied again for covering the surface of the diced joined layered body with the primer treatment film 18.

S7 : Next is to fix the primer-treated joined layered body to a mold 21, followed by pouring a resin precursor, which covers parts of the acoustic lens, groove filling, and external surface, and curing the resin precursor

(refer to Figs. 11D and 11E). The resin precursor comprises a silicone elastomer precursor and a dilute solvent. A product named "ELASTOSIL" produced by Wacker Asahikasei Silicone Co., Ltd. is used as the silicone elastomer (i.e., the silicone resin), while a product named "ISOPAR E" (sold by Exxon Mobile Chemicals) is used as the dilute solvent. The above described process forms the acoustic lens / external cover 2.

Here, the resin precursor cures in two hours under the condition of 55°C. In this event, added is a dilute fluid (i.e., a solvent of an aromatic series) so that the cured resin possesses an adequate degree of viscosity. A use of the dilute fluid, however, leaves a residual odor after the curing. A further post-curing treatment for 36 hours at 85°C is effective for eliminating the residual odor.

Note that the silicone resin of the gradient material may be formed in the S7 as described for the fourth embodiment. In this case, pouring an acoustic lens precursor, which is dispersed with hollow particulates, into the silicone resin, followed by turning it upside down by the mold 21 per se (i.e., making in the state of Fig. 11D) causes the hollow particulates to rise toward the deeper part of the groove by the effect of specific gravity.

Also, in order to accomplish the third embodiment, the process of S7 may be followed by further forming a nano-coating film layer on the surface of the acoustic lens / external cover 2, thereby making it as the third embodiment.

As described above, covering the above configured structure body with the acoustic lens / external cover 2 makes it possible to produce the array type ultrasonic transducer according to the present invention.

Note that the first through sixth embodiment may be combined in any manner depending on the use. Also note that, while the first through seventh embodiments use two acoustic matching layers, (i.e., the first and second acoustic matching layers), there may be one or two or more acoustic matching layers, for example, in lieu of being limited by the embodiments.

As described thus far, a use of the present invention enables the integrated forming of the acoustic lens material and groove filling materials by using the same material, thus eliminating a use of a specific adhesive. This accordingly makes it possible to prevent a variation in characteristic of an ultrasonic wave by an excessive amount of adhesive otherwise flowing unevenly in between elements. It also makes it possible to prevent a crosstalk and improve the mechanical strength so as to prevent an element from leaning over.

## Claims

1. An ultrasonic transducer (1) comprising a plurality of resonator elements, each including a piezoelectric resonator (5) oscillating to cause the emission of an ultrasonic wave and one or more acoustic matching layer(s) (6, 7), and an acoustic lens (201),
**characterized in that**
a gap area between adjacent resonator elements is filled with the same constituent material as that of the acoustic lens (201).

2. The ultrasonic transducer (1) according to claim 1, wherein
an outer surface of the ultrasonic transducer (1) is covered with the same constituent material as that of said acoustic lens (201).

3. The ultrasonic transducer (1) according to claim 1, wherein
a constituent material of said acoustic lens (201) is a gradient material.

4. The ultrasonic transducer (1) according to claim 3, wherein
said gradient material is one dispersing inorganic fine particulate powder in a silicone resin with a filling density of the inorganic fine particulate powder becoming lower with the direction from an oscillation generation surface of said piezoelectric element to the bordering surface between said acoustic lens (201) and acoustic matching layer (6, 7).

5. The ultrasonic transducer (1) according to claim 4, wherein
said inorganic fine particulate powder includes at least one of tungsten, tungsten oxide, aluminum oxide and zirconium oxide.

6. The ultrasonic transducer (1) according to claim 3, wherein
particulates, each of which possesses a hollow structure and a smaller specific gravity than a silicone resin, are dispersed in the silicone resin for said gradient material with a filling density of the inorganic fine particulate powder decreasing with the direction from an oscillation generation surface of said piezoelectric element to the bordering surface between said acoustic lens (201) and acoustic matching layer (6, 7).

7. The ultrasonic transducer (1) according to claim 6, wherein
said particulate is constituted of a glass material.

8. The ultrasonic transducer (1) according to claim 6, wherein
said particulate is constituted of a polymer material.

9. The ultrasonic transducer (1) according to claim 2, wherein
a thin film layer having a corrosion resistance property or humidity resistance property intervenes between a surface where a material, which forms said acoustic lens (201) and fills between said oscillation elements, and the oscillation element contact with each other.

10. The ultrasonic transducer (1) according to claim 9, wherein
said thin film layer includes a nano-coating layer containing an inorganic compound component.

11. The ultrasonic transducer (1) according to claim 10, wherein
said inorganic compound component includes at least either one of silicone, titanium, or zirconium.

12. The ultrasonic transducer (1) according to claim 2, wherein
a thin film layer having a corrosion resistance property or humidity resistance property exists on a covered surface of said ultrasonic transducer which is covered with the same constituent material as that of said acoustic lens (201).

13. The ultrasonic transducer (1) according to claim 12, wherein
said thin film layer includes a nano-coating film containing an inorganic compound component.

14. The ultrasonic transducer (1) according to claim 13, wherein
said nano-coating film contains at least either one of silicone oxide, titanium oxide, or zirconium oxide.

15. The ultrasonic transducer (1) according to claim 14, forming a silver nano-coating film on a surface of said thin film layer.

16. A production method for an array type ultrasonic transducer (1), comprising:
a transducer element forming process for forming a transducer element comprising a plurality of resonator elements, each including a piezoelectric resonator (5) for emitting an ultrasonic wave and one or more acoustic matching layers (6, 7), and an acoustic lens (201); and
a resin precursor curing process for curing a resin precursor by fixing a layered body (5, 6, 7, 9), which is obtained by the transducer element forming process, to a preconfigured mold and making the layered body (5, 6, 7, 9) contact with the resin precursor which covers parts of the acoustic lens (201), and filling material in between the resonator elements and an outside of the layered body (5, 6, 7, 9),
**characterized in that**
a gap area between adjacent resonator elements is filled with the same constituent material as that of the acoustic lens (201).

17. The production method for an array type ultrasonic transducer (1) according to claim 16, further comprising:
a nano-coating film layer forming process for forming a nano-coating film layer on the cured resin precursor after the resin precursor curing process.

18. The production method for an array type ultrasonic transducer (1) according to claim 16, wherein
said resin precursor contains a silicone elastomer precursor and a dilute solvent.

19. An ultrasonic wave endoscope apparatus comprising the array type ultrasonic transducer (1) according to claim 1.

20. An ultrasonic wave endoscope apparatus comprising an array type ultrasonic transducer (1) produced by the production method according to claim 16.

## Patentansprüche

1. Ultraschallwandler (1) mit mehreren Resonatorelementen, wobei jedes einen piezoelektrischen Resonator (5), welcher schwingt, um die Emission einer Ultraschallwelle zu bewirken, und eine oder mehrere akustische Anpassungsschichten (6, 7) und eine akustische Linse (201) einschließt,
**dadurch gekennzeichnet, dass**
ein Lückenbereich zwischen benachbarten Resonatorelementen mit demselben Bestandteilsmaterial wie demjenigen der akustischen Linse (201) gefüllt ist.

2. Ultraschallwandler (1) nach Anspruch 1, wobei
eine Außenfläche des Ultraschallwandlers (1) mit demselben Bestandteilsmaterial wie demjenigen der akustischen Linse (201) bedeckt ist.

3. Ultraschallwandler (1) nach Anspruch 1, wobei
ein Bestandteilsmaterial der akustischen Linse (201) ein Gradientenwerkstoff ist.

4. Ultraschallwandler (1) nach Anspruch 3, wobei
der Gradientenwerkstoff ein dispergierendes anorganisches feines partikelförmiges Pulver in einem Silikonharz ist, wobei die Fülldichte des anorganischen feinen partikelförmigen Pulvers mit der Richtung von einer Schwingungserzeugungsfläche des piezoelektrischen Elements zur Grenzfläche zwischen der akustischen Linse (201) und der akustischen Anpassungsschicht (6, 7) niedriger wird.

5. Ultraschallwandler (1) nach Anspruch 4, wobei
das anorganische feine partikelförmige Pulver Wolfram und/oder Wolframoxid und/oder Aluminiumoxid und/oder Zirkoniumoxid enthält.

6. Ultraschallwandler (1) nach Anspruch 3, wobei
Partikel, von denen jedes eine Hohlstruktur und ein kleineres spezifisches Gewicht als ein Silikonharz besitzt, in dem Silikonharz für den Gradientenwerkstoff dispergiert sind, wobei die Fülldichte des anorganischen feinen partikelförmigen Pulvers mit der Richtung von einer Schwingungserzeugungsfläche des piezoelektrischen Elements zur Grenzfläche zwischen der akustischen Linse (201) und der akustischen Anpassungsschicht (6, 7) abnimmt.

7. Ultraschallwandler (1) nach Anspruch 6, wobei
das Partikelmaterial aus einem Glasmaterial besteht.

8. Ultraschallwandler (1) nach Anspruch 6, wobei
das Partikelmaterial aus einem Polymermaterial besteht.

9. Ultraschallwandler (1) nach Anspruch 2, wobei
eine Dünnfilmschicht mit einer Korrosionsbeständigkeitseigenschaft oder Feuchtigkeitsbeständigkeitseigenschaft zwischen eine Oberfläche kommt, an der ein Material, das die akustische Linse (201) bildet und zwischen den Schwingungselementen füllt, und das Schwingungselement einander berühren.

10. Ultraschallwandler (1) nach Anspruch 9, wobei
die Dünnfilmschicht eine Nanoüberzugsschicht einschließt, die eine anorganische Verbindungskomponente enthält.

11. Ultraschallwandler (1) nach Anspruch 10, wobei
die anorganische Verbindungskomponente Silikon und/oder Titan und/oder Zirkonium enthält.

12. Ultraschallwandler (1) nach Anspruch 2, wobei
eine Dünnfilmschicht mit einer Korrosionsbeständigkeitseigenschaft oder Feuchtigkeitsbeständigkeitseigenschaft auf einer bedeckten Oberfläche des Ultraschallwandlers vorhanden ist, die mit demselben Bestandteilsmaterial wie demjenigen der akustischen Linse (201) bedeckt ist.

13. Ultraschallwandler (1) nach Anspruch 12, wobei
die Dünnfilmschicht einen Nanoüberzugsfilm einschließt, der eine anorganische Verbindungskomponente enthält.

14. Ultraschallwandler (1) nach Anspruch 13, wobei
der Nanoüberzugsfilm Silikonoxid und/oder Titanoxid und/oder Zirkoniumoxid enthält.

15. Ultraschallwandler (1) nach Anspruch 14, der einen Silbernanoüberzugsfilm auf einer Oberfläche der Dünnfilmschicht bildet.

16. Herstellungsverfahren für einen Ultraschallwandler (1) vom Array-Typ, mit:
einem Wandlerelement-Bildungsvorgang zum Bilden eines Wandlerelements mit mehreren Resonatorelementen, wobei jedes einen piezoelektrischen Resonator (5) zum Emittieren einer Ultraschallwelle und eine oder mehrere akustische Anpassungsschichten (6, 7) und eine akustische Linse (201) enthält; und
einem Harzvorläufer-Aushärtungsvorgang zum Aushärten eines Harzvorläufers durch Befestigen eines geschichteten Körpers (5, 6, 7, 9), der durch den Wandlerelement-Bildungsvorgang erhalten wird, auf einer vorab ausgebildeten Form und Inberührungbringen des geschichteten Körpers (5, 6, 7, 9) mit dem Harzvorläufer, der Teile der akustischen Linse (201) bedeckt, und Einfüllen von Material zwischen die Resonatorelemente und die Außenseite des geschichteten Körpers (5, 6, 7, 9),
**dadurch gekennzeichnet, dass**
ein Lückenbereich zwischen benachbarten Resonatorelementen mit demselben Bestandteilsmaterial wie demjenigen der akustischen Linse (201) gefüllt ist.

17. Herstellungsverfahren für einen Ultraschallwandler (1) vom Array-Typ nach Anspruch 16, ferner mit:
einem Nanoüberzugsfilmschicht-Bildungsvorgang zum Bilden einer Nanoüberzugsfilmschicht auf dem ausgehärteten Harzvorläufer nach dem Harzvorläufer-Aushärtungsvorgang.

18. Herstellungsverfahren für einen Ultraschallwandler (1) vom Array-Typ nach Anspruch 16, wobei
der Harzvorläufer einen Silikonelastomervorläufer und ein verdünntes Lösungsmittel enthält.

19. Ultraschallwellen-Endoskopvorrichtung mit dem Ultraschallwandler (1) vom Array-Typ nach Anspruch 1.

20. Ultraschallwellen- Endoskopvorrichtung mit einem Ultraschallwandler (1) vom Array-Typ, der durch das Herstellungsverfahren nach Anspruch 16 hergestellt wurde.

## Revendications

1. Transducteur à ultrasons (1) comprenant une pluralité d'éléments résonateurs, comprenant chacun un résonateur piézo-électrique (5) oscillant pour provoquer l'émission d'une onde ultrasonore et une ou plusieurs couches d'adaptation acoustique (6, 7), et une lentille acoustique (201),
**caractérisé en ce que**
une zone d'espacement entre les éléments résonateurs adjacents est remplie avec la même matière constitutive que celle de la lentille acoustique (201).

2. Transducteur à ultrasons (1) selon la revendication 1, dans lequel
une surface externe du transducteur à ultrasons (1) est recouverte avec la même matière constitutive que celle de ladite lentille acoustique (201).

3. Transducteur à ultrasons (1) selon la revendication 1, dans lequel
une matière constitutive de ladite lentille acoustique (201) est un matériau à gradient.

4. Transducteur à ultrasons (1) selon la revendication 3, dans lequel
ledit matériau à gradient est une poudre de fines particules inorganiques dispersante dans une résine de silicium avec une densité de remplissage de la poudre de fines particules inorganiques devenant plus faible avec la direction allant d'une surface de génération d'oscillation dudit élément piézo-électrique à la surface de bordure entre ladite lentille acoustique (201) et la couche d'adaptation acoustique (6, 7).

5. Transducteur à ultrasons (1) selon la revendication 4, dans lequel
ladite poudre de fines particules inorganiques comprend au moins l'un parmi du tungstène, de l'oxyde de tungstène, de l'oxyde d'aluminium et de l'oxyde de zirconium.

6. Transducteur à ultrasons (1) selon la revendication 3, dans lequel
des particules, dont chacune possède une structure creuse et une gravité spécifique inférieure à une résine de silicium, sont dispersées dans la résine de silicium pour ledit matériau à gradient avec une densité de remplissage de la poudre de fines particules inorganiques diminuant avec la direction allant d'une surface de génération d'oscillation dudit élément piézo-électrique à la surface de bordure entre ladite lentille acoustique (201) et la couche d'adaptation acoustique (6, 7).

7. Transducteur à ultrasons (1) selon la revendication 6, dans lequel ladite particule est constituée d'un matériau de verre.

8. Transducteur à ultrasons (1) selon la revendication 6, dans lequel ladite particule est constituée d'un matériau polymère.

9. Transducteur à ultrasons (1) selon la revendication 2, dans lequel
une couche de film mince possédant une propriété de résistance à la corrosion ou une propriété de résistance à l'humidité s'interpose entre une surface sur laquelle un matériau qui forme ladite lentille acoustique (201) et remplit l'espace entre lesdits éléments d'oscillation, et l'élément d'oscillation se contactent l'un l'autre.

10. Transducteur à ultrasons (1) selon la revendication 9, dans lequel
ladite couche de film mince comprend une couche à nano-revêtement contenant un composant de composé inorganique.

11. Transducteur à ultrasons (1) selon la revendication 10, dans lequel
ledit composant de composés inorganiques comprend au moins l'un ou l'autre parmi du silicium, du titane ou du zirconium.

12. Transducteur à ultrasons (1) selon la revendication 2, dans lequel
une couche de film mince possédant une propriété de résistance à la corrosion ou une propriété de résistance à l'humidité se trouve sur une surface couverte dudit transducteur à ultrasons qui est couvert de la même matière constitutive que celle de ladite lentille acoustique (201).

13. Transducteur à ultrasons (1) selon la revendication 12, dans lequel
ladite couche de film mince comprend un film de nano-revêtement contenant un composant de composé inorganique.

14. Transducteur à ultrasons (1) selon la revendication 13, dans lequel
ledit film de nano-revêtement contient au moins l'un ou l'autre parmi de l'oxyde de silicium, de l'oxyde de titane ou de l'oxyde de zirconium.

15. Transducteur à ultrasons (1) selon la revendication 14, formant un film de nano-revêtement d'argent sur une surface de ladite couche de film mince.

16. Procédé de production pour un transducteur à ultrasons de type matrice (1), comprenant :
un processus de formation de l'élément d'un transducteur destiné à former un élément de transducteur comprenant une pluralité d'éléments résonateurs, comprenant chacun un résonateur piézo-électrique (5) destiné à émettre une onde ultrasonore et une ou plusieurs couches d'adaptation acoustique (6, 7), et une lentille acoustique (201) ; et
un processus de durcissement de précurseur de résine destiné à durcir un précurseur de résine en fixant un corps stratifié (5, 6, 7, 9) qui est obtenu par le processus de formation de l'élément de transducteur, sur un moule pré-configuré et amenant le corps stratifié (5, 6, 7, 9) à contacter le précurseur de résine qui couvre les parties de la lentille acoustique (201), et destiné à remplir un matériau entre les éléments résonateurs et un extérieur du corps stratifié (5, 6, 7, 9),
**caractérisé en ce que**
une zone d'espacement entre les éléments résonateurs adjacents est remplie avec la même matière constitutive que celle de la lentille acoustique (201).

17. Procédé de production pour un transducteur à ultrasons de type matrice (1) selon la revendication 16, comprenant en outre :
un processus de formation de couche de film de nano-revêtement destiné à former une couche de film de nano-revêtement sur le précurseur de résine durci après le processus de durcissement de précurseur de résine.

18. Procédé de production pour un transducteur à ultrasons de type matrice (1) selon la revendication 16, dans lequel
ledit précurseur de résine contient un précurseur d'élastomère de silicone et un solvant dilué.

19. Appareil d'endoscope à onde ultrasonore comprenant le transducteur à ultrasons de type matrice (1) selon la revendication 1.

20. Appareil d'endoscope à onde ultrasonore comprenant un transducteur à ultrasons de type matrice (1) produit par le procédé de production selon la revendication 16.
